Europäisches Patentamt

(19) European Patent Office

Office européen des brevets

(11) Numéro de publication: **0 062 578**
**B1**

(12) **FASCICULE DE BREVET EUROPEEN**

(45) Date de publication du fascicule du brevet:
**20.06.84**

(21) Numéro de dépôt: **82400578.9**

(22) Date de dépôt: **30.03.82**

(51) Int. Cl.³: **A 61 K 33/10,** A 61 K 33/08,
A 61 K 33/06, A 61 K 33/00,
A 61 K 35/02 // (A61K35/02,
33/10, 33/08, 33/06, 33/00)

(54) **Composition thérapeutique utile comme topique digestif.**

(30) Priorité: **01.04.81 FR 8106560**

(43) Date de publication de la demande:
**13.10.82 Bulletin 82/41**

(45) Mention de la délivrance du brevet:
**20.06.84 Bulletin 84/25**

(84) Etats contractants désignés:
**BE CH DE FR GB IT LI LU NL SE**

(56) Documents cités:
**FR - M - 781**
**GB - A - 1 206 870**
**US - A - 2 477 080**

**DICTIONNAIRE VIDAL, 55e Edition, 1979, PARIS (FR)**
**page 659-660, "DOPS"**
**DICTIONNAIRE VIDAL, 55e Edition, 1979 PARIS (FR)**
**page 1213 "LYANTIL" (Adultes, Enfants)**

(73) Titulaire: **LABORATOIRES HUMAN-PHARM S.A. Société**
**Anonyme dite:, 94, rue Edouard-Vaillant,**
**F-92300 Levallois-Perret (FR)**

(72) Inventeur: **Bodin, Jacques, 42 rue du Président Wilson,**
**F-92300 Levallois (FR)**
Inventeur: **Chardin, Arlette, 46 rue Gabriel Péri,**
**F-92300 Levallois-Perret (FR)**

(74) Mandataire: **Combe, André et al, CABINET BEAU DE**
**LOMENIE 55 rue d'Amsterdam, F-75008 Paris (FR)**

ACTORUM AG

## Description

La présente invention concerne une nouvelle composition thérapeutique douée de propriétés couvrantes et thixotropiques et qui est utile comme topique digestif, notamment dans le domaine des pansements gastriques.

Selon l'invention, on préconise une composition thérapeutique constituée par une association synergétique d'au moins deux substances minérales, utile comme topique digestif et offrant l'avantage de pouvoir être administrée dans les cas de diabète et de régime.

On connaît notamment du Dictionnaire Vidal, 55e édition (OVP, Paris, 1979), pages 659–660, une spécialité renfermant, sous forme de poudre, 2% en poids de $TiO_2$ et 95% en poids de produits anti-acides, et, sous forme de comprimé, 0,63% en poids de $TiO_2$ et 98,10% en poids de produits anti-acides. Le $TiO_2$ utilisé dans cette spécialité est présenté comme agent protecteur de la muqueuse gastrique.

On a trouvé de façon surprenante que pour avoir un bon pouvoir couvrant pendant une longue durée de temps, et d'excellentes propriétés thixotropiques, il est important que la teneur en $TiO_2$ dans la composition thérapeutique soit supérieure ou égale à 5% en poids par rapport au poids de ladite composition.

La composition selon l'invention est en particulier applicable dans le traitement et la prévention des ulcères gastriques, oesophagites, hernies hiatales, reflux gastro-oesophagien, gastrites, ulcères duodénaux, duodénites, entérites, entérocolites, colites, colopathies, rectites et diarrhées aigues d'origine bactérienne ou virale, tant chez l'adulte que chez l'enfant. Elle favorise la régénération des muqueuses et, en raison de ses propriétés couvrantes, autorise la cicatrisation de l'épithélium qu'elle protège.

Cette composition est caractérisée en ce qu'elle renferme, en association avec un excipient physiologiquement acceptable, au moins deux substances minérales en tant qu'ingrédients actifs:

A – l'oxyde de titane en tant que moyen couvrant et thixotrope en suspension, et
B – un moyen antiacide, choisi parmi l'ensemble constitué par $CaCO_3$, $MgO$, $MgCO_3$, $Al_2O_3$, $Al(OH)_3$, le phsophate d'aluminium et $BaCO_3$,

le rapport pondéral des moyens A – B étant compris entre (1:10) et (10:1), et la teneur en moyen A étant supérieure ou égale à 5% en poids par rapport au poids de ladite composition.

Au niveau de l'estomac, le moyen A agit sur le plan mécanique en tant qu'agent couvrant et isolant de la muqueuse par rapport au contenu stomacal et sur le plan thérapeutique en tant qu'agent favorisant la régénération de la muqueuse et permettant la cicatrisation de l'épithélium, et le moyen B intervient par son pouvoir antiacide. Les moyens A et B agissent de la même façon sur les autres muqueuses du système digestif. Le moyen A préféré est la variété anatase de $TiO_2$ et le moyen B préféré est $CaCO_3$. Le rapport pondéral A–B préféré est compris entre (1:1) et (10:1).

De façon avantageuse la composition thérapeutique selon l'invention peut renfermer en outre une charge minérale C ou un agent gélifiant D. De préférence on fera appel à une composition renfermant les 4 moyens A, B, C et D.

Le moyen C est un agent favorisant le transit intestinal et qui a un effet synergétique avec l'ensemble A et B sur le pouvoir couvrant et protecteur, d'une part, et les propriétés thixotropiques, d'autre part. Le rapport pondéral C–A est compris entre (1:90) et (1:10) et avantageusement entre (1:50) et (1:20). De façon avantageuse, le moyen C sera choisi parmi l'ensemble constitué par l'argile, la bentonite et la montmorillonite. On a observé à ce sujet que le talc et le mica (substances qui pourraient être intéressantes en raison de leur propriété lubrifiante) ne conviennent pas en tant que moyen C dès lors que leur utilisation dans la composition selon l'invention diminue les propriétés thixotropiques.

Le moyen gélifiant D que l'on préconise sera avantageusement choisi parmi l'ensemble constitué par les pectines et les dérivés cellulosiques, notamment les éthers de cellulose et la carboxyméthylcellulose. Le rapport pondéral D–A préféré est compris entre (1:50) et (1:20).

La composition selon l'invention peut être administrée en thérapeutique humaine et vétérinaire, notamment sous forme de comprimés, de capsules, de suspensions aqueuses et de gels, par voie orale, et, le cas échéant, sous forme de suppositoires, par voie rectale.

D'autres avantages et caractéristiques de l'invention seront mieux compris à la lecture qui va suivre d'exemples de réalisation nullement limitatifs, mais donnés à titre d'illustration. L'exemple 1 ci-après constitue le meilleur mode de mise en oeuvre.

Exemple 1

On prépare une composition utile comme topique digestif, à partir des moyens A à D et des excipients donnés dans la formulation suivante:

Formulation 1

| | | |
|---|---|---|
| moyen A: $TiO_2$ (anatase) | 9 | g |
| moyen B: $CaCO_3$ | 3 | g |
| moyen C: argile | 0,25 | g |
| moyen D: carboxyméthylcellulose | 0,30 | g |
| excipients: glycérine | 20 | g |
| saccharinate de sodium | 0,025 | g |
| p-hydroxybenzoate de méthyle | 0,13 | g |
| p-hydroxybenzoate de propyle | 0,02 | g |
| arôme citron | 0,08 | g |
| arôme framboise | 0,168 | g |
| Colorant (Rouge 2G à 1% poids/volume) | 0,2 | g |
| eau distillée, q.s.p. | 100 | g |

Dans de l'eau distillée, on introduit successivement (i) le saccharinate de sodium, (ii) le parahydroxybenzoate de méthyle puis le parahydroxybenzoate de propyle, (iii) la glycérine, (iv) le moyen D, la CMC, (v) le moyen C, argile commercialisée sous le nom de «VEEGUM HV», (vi) le moyen B, le carbonate de calcium, (vii) le moyen A, l'oxyde de titane, (viii) l'arôme citron, commercialisé sous la nomenclature de «GIVAUDAN 60863-76», l'arôme framboise, commercialisé sous la nomenclature de «IFF 6K 103» et le colorant, puis (ix) de l'eau distillée en quantité suffisante pour obtenir une composition d'un poids total de 100 g qui est notamment présentée sous forme de gel.

Exemple 2

On prépare selon les modalités opératoires décrites à l'exemple 1, une composition utile comme topique digestif selon l'invention dans laquelle TiO$_2$ (moyen A), CaCO$_3$ (moyen B), bentonite (moyen C) et pectine (moyen D) sont dans le rapport pondéral (30:30:1:1).

Exemple 3

Comme indiqué à l'exemple 2, on prépare une composition utile comme topique digestif selon l'invention dans laquelle le rapport pondéral TiO$_2$ – CaCO$_3$ – argile – CMC est de (90:20:2:3).

Exemples 4 à 9

En procédant comme indiqué à l'exemple 1, mais en remplaçant dans la formulation les 3 g de CaCO$_3$ par 4 g de MgO, 10 g de phosphate d'aluminium, 5 g de Al$_2$O$_3$, 4 g de MgCO$_3$, 4 g de BaCO$_3$ et respectivement 5 g de Al(OH)$_3$, on obtient des compositions (exemples 4 à 9) selon l'invention utiles comme topiques digestifs.

Exemple 10

Selon les modalités opératoires décrites à l'exemple 1, mais en utilisant dans la formulation une quantité de TiO$_2$ de 5 g au lieu de 9 g, on obtient une composition utile comme topique digestif et ayant un excellent pouvoir couvrant (voir tableau 1 ci-après).

Exemple Comparatif

Selon les modalités opératoires décrites à l'exemple 1, mais en utilisant dans la formulation une quantité de TiO$_2$ de 4,9 g au lieu de 9 g on obtient une composition (désignée ci-après A3) qui n'a pas un bon pouvoir couvrant quatre heures après administration (voir tableau 1 ci-après).

On a résumé ci-après une partie des essais qui on été entrepris avec des compositions selon l'invention, en ce qui concerne leur pouvoir couvrant.

Le principe de la recherche d'un pouvoir couvrant repose sur la connaissance que, chez le rat soumis à un jeûne prolongé, l'administration d'un topique digestif, en tant que pansement gastrique, entraîne un dépôt médicamenteux que l'on peut apprécier après sacrifice des animaux et étalement des estomacs. Selon deux temps de mesure, le sacrifice des animaux intervenant 0,5 h et 4 h après administration:

110 rats mâles Wistar pesant chacun 300 g sont mis à jeun pendant 48 h, l'eau de boisson étant laissée ad libitum, puis répartis en lots comme suit: 2 lots témoins (de 5 animaux chacun) et 2 lots de 10 animaux chacun pour chaque composition testée, à savoir les exemples 1 et 10 selon l'invention, un topique digestif de référence (A1) renfermant dans sa formulation 55% en poids de phosphate d'aluminium colloïdal, la spécialité (A2) décrite dans le Dictionnaire Vidal sus-visé, et l'exemple comparatif (A3).

Tous les animaux reçoivent 5 ml d'eau par tubage gastrique, 30 min avant l'administration des compositions à étudier afin d'éliminer tout résidu stomacal. Lesdites compositions sont administrées par tubage gastrique sous un volume de 1 ml pour 100 g de poids corporel, les lots témoins étant traités dans le mêmes conditions avec de l'eau ordinaire. Le sacrifice des animaux intervient 0,5 h et 4 h après l'administration.

Après sacrifice (rupture de la nuque), les estomacs sont prélevés et fendus suivant la grande courbure. Ils sont nettoyés de l'excédent éventuel de composition (cas de la mesure à 0,5 h) par cinq immersions successives dans un soluté aqueux isotonique de NaCl. Ils sont alors étalés et le pouvoir couvrant est apprécié par l'intensité du dépôt médicamenteux au niveau du rumen (R) et au niveau de la partie glandulaire (G) de l'estomac, selon la cotation suivante:

O: absence de dépôt,
1: dépôts ponctuels,
2: dépots nets,
3: dépots importants,
4: dépots généralisés.

Les résultats consignés dans le tableau 1 ci-après (qui donne les sommes des scores individuels, selon la cotation sus-décrite), montrent que: (i) après 0,5 h les compositions des exemples 1 et 10 et la composition de référence A1 ont approximativement le même pouvoir couvrant (chaque composition étant encore en partie présente dans l'estomac) et (ii) après 4 h, les compositions selon les exemples 1 et 10 ont un pouvoir couvrant supérieur à celui des compositions A1, A2, et A3.

Tableau 1
Pouvoir couvrant

| Composition | Teneur en $TiO_2$ | 0,5 h après administration | | | 4 h après administration | | |
|---|---|---|---|---|---|---|---|
| | | R | G | R + G | R | G | R + G |
| eau (témoin) | 0% | 0 | 0 | 0 | 0 | 0 | 0 |
| A1 | 0% | 35 | 18 | 53 | 9 | 8 | 17 |
| A2 | 2% | 29 | 16 | 45 | 8 | 6 | 14 |
| A3 | 4,9% | 30 | 17 | 47 | 8 | 7 | 15 |
| Ex 1 | 9% | 31 | 21 | 52 | 13 | 15 | 28 |
| Ex 10 | 5% | 31 | 20 | 51 | 13 | 14 | 27 |

Notes:

R: rumen
G: partie glandulaire
R+G: somme de R et G
A1: composition de référence ayant la formulation suivante pour 100 g:

- phosphate d'aluminium colloïdal    55   g
- parahydroxybenzoate de méthyle    0,11   g
- parahydroxybenzoate de propyle    0,04   g
- acide sorbique    0,15   g
- saccharose    15   g
- autre excipients:
  pectine, agar-agar, essence d'orange et eau, q.s.p.    100   g

A2: composition de référence décrite dans le Dictionnaire Vidal susvisé et ayant la formulation suivante pour 100 g (présentation poudre):

- $NaHCO_3$    52   g
- $CaCO_3$    41   g
- $Ca_3(PO_4)_2$    2   g
- $Mg(OH)_2$    3   g
- $TiO_2$    2   g

A3: exemple comparatif

## Revendications

1. Composition thérapeutique utile comme topique digestif comprenant comme ingrédients actifs de substances minérales, et douée de propriétés couvrantes et thixotropiques, caractérisée en qu'elle renferme, en association avec un excipient physiologiquement acceptable, au moins deux substances minérales:

A – l'oxyde de titane en tant que moyen couvrant et thixotrope en suspension, et
B – un moyen anti-acide, choisi parmi l'ensemble constitué par $CaCO_3$, MgO, le phosphate d'aluminium, $Al_2O_3$, $Al(OH)_3$, $MgCO_3$ et $BaCO_3$,

le rapport pondéral A–B étant compris entre (1:10) et (10:1), et la teneur en moyen A étant supérieure ou égale à 5% en poids par rapport au poids de ladite composition.

2. Composition selon la revendication 1, caractérisée en ce que, outre les moyens A et B, elle renferme un moyen C favorisant le transit intestinal et choisi parmi l'ensemble constitué par l'argile, la bentonite et la montmorillonite, le rapport pondéral C–A étant compris entre (1:90) et (1:10) et avantageusement entre (1:50) et (1:20).

3. Composition selon la revendication 1, caractérisée en que, outre les moyens A et B, elle renferme un moyen gélifiant D, choisi parmi la pectine et les dérivés de cellulose, le rapport pondéral D–A étant compris entre (1:50) et (1:20).

4. Composition selon la revendication 1, caractérisée en ce qu'elle renferme:

A – de l'oxyde de titane à une concentration supérieure ou égale à 5% en poids par rapport au poids de ladite composition,
B – un moyen anti-acide, choisi parmi l'ensemble constitué par $CaCO_3$, MgO, le phosphate d'aluminium, $Al_2O_3$, $Al(OH)_3$, $MgCO_3$ et $BaCO_3$, le rapport pondéral A–B étant compris entre (1:10) et (10:1), et de préférence entre (1:1) et (10:1),
C – un moyen favorisant le transit intestinal choisi parmi l'ensemble constitué par l'argile, la bentonite et la montmorillonite, le rapport pondéral C – A étant compris entre (1:50) et (1:20), et
D – un moyen gélifiant choisi parmi l'ensemble constitué par la pectine et la carboxyméthylcellulose, le rapport pondéral D–A étant compris entre (1:50) et (1:20).

5. Composition selon la revendication 4, caractérisée en ce qu'elle renferme en pourcentage en poids par rapport au poids de ladite composition 9% de $TiO_2$, 3% de $CaCo_3$, 0,25% d'argile et 0,30% de carboxyméthylcellulose.

## Patentansprüche

1. Als verdauungsförderndes topisches Mittel geeignete therapeutische Zusammensetzung mit Mineralstoffen als aktive Ingredienzien, die mit umhüllenden und thixotropen Eigenschaften versehen ist, dadurch gekennzeichnet, dass sie in Verbindung mit einem physiologisch akzeptablen Exzipienten zumindest zwei Mineralstoffe enthält:

A – Titanoxid als Umhüllungs- und thixotropes Mittel in Suspension und
B – ein antacides Mittel, ausgewählt aus der Gruppe bestehend aus $CaCO_3$, MgO, Aluminiumphosphat, $Al_2O_3$, $Al(OH)_3$, $MgCO_3$ und $BaCO_3$,

wobei das Gewichtsverhältnis A–B zwischen (1:10) und (10:1) beträgt und der Gehalt am Mittel A höher oder gleich 5 Gew.-%, bezogen auf das Gewicht der Zusammensetzung, ausmacht.

2. Zusammensetzung nach Anspruch 1, dadurch gekennzeichnet, dass sie neben den Mitteln A und B ein Mittel C enthält, das den Darmdurchgang begünstigt und ausgewählt ist aus der Gruppe bestehend aus Ton, Bentonit und Montmorillonit, wobei das Gewichtsverhältnis C–A zwischen (1:90) und (1:10), vorteilhaft zwischen (1:50) und (1:20) beträgt.

3. Zusammensetzung nach Anspruch 1, dadurch gekennzeichnet, dass sie neben den Mitteln A und B ein gelbildendes Mittel D, ausgewählt aus Pectin und Cellulosederivaten, enthält, wobei das Gewichtsverhältnis D–A zwischen (1:50) und (1:20) beträgt.

4. Zusammensetzung nach Anspruch 1, dadurch gekennzeichnet, dass sie enthält:

A – Titanoxid in einer Konzentration höher oder gleich 5 Gew.-%, bezogen auf das Gewicht der Zusammensetzung,
B – ein antacides Mittel, ausgewählt aus der Gruppe bestehend aus $CaCO_3$, MgO, Aluminiumphosphat, $Al_2O_3$, $Al(OH)_3$, $MgCO_3$ und $BaCO_3$, wobei das Gewichtsverhältnis A–B zwischen (1:10) und (10:1), vorzugsweise zwischen (1:1) und (10:1) beträgt,
C – ein den Darmdurchgang begünstigendes Mittel, ausgewählt aus der Gruppe bestehend aus Ton, Bentonit und Montmorillonit, wobei das Gewichtsverhältnis C–A zwischen (1:50) und (1:20) beträgt, und
D – ein gelbildendes Mittel, ausgewählt aus der Gruppe bestehend aus Pectin und Carboxymethylcellulose, wobei das Gewichtsverhältnis D–A zwischen (1:50) und (1:20) beträgt.

5. Zusammensetzung nach Anspruch 4, dadurch gekennzeichnet, dass sie in Gewichtsprozent, bezogen auf das Gewicht der Zusammensetzung, 9% $TiO_2$, 3% $CaCO_3$, 0,25% Ton und 0,30% Carboxymethylcellulose enthält.

## Claims

1. Therapeutical composition useful as digestive topic, comprising as active ingredient mineral substances, and endowed with coating and thixotropic properties, characterized in that it contains, in association with a physiologically acceptable excipient, at least two mineral substances:

A – titanium oxide as coating and thixotropic means in suspension, and
B – an antacid means, selected from the group constituted by $CaCO_3$, MgO, aluminium phosphate, $Al_2O_3$, $Al(OH)_3$, $MgCO_3$ and $BaCO_2$,

the weight ratio A–B being between (1:10) and (10:1), and the content of means A being greater than or equal to 5% by weight with respect to the weight of said composition.

2. Composition according to claim 1, characterized in that in addition to means A and B, it contains a means C promoting the intestinal transit and selected from the group constituted by clay, bentonite and montmorillonite, the weight ratio C–A being between (1:90) and (1:10) and advantageously between (1:50) and (1:20).

3. Composition according to claim 1, characterized in that, in addition to means A and B, it contains a gelling means D, selected from pectine and cellulose derivatives, the weight ratio D–A being between (1:50) and (1:20).

4. Composition according to claim 1, characterized in that it contains:

A – titanium oxide at a concentration greater than or equal to 5% by weight with respect to the weight of said composition,
B – an antacid means, selected from the group constituted by $CaCO_3$, MgO, aluminium phosphate, $Al_2O_3$, $Al(OH)_3$, $MgCO_3$ and $BaCO_3$, the weight ratio A–B being between (1:10) and (10:1), and preferably between (1:1) and (10:1),
C – a means promoting intestinal transit selected from the group constituted by clay, bentonite and montmorillonite, the weight ratio C–A being between (1:50) and (1:20), and
D – a gelling means selected from the group constituted by pectine and carboxymethylcellulose, the weight ratio D–A being between (1:50) and (1:20).

5. Composition according to claim 4, characterized in that it contains, in percentage by weight with respect to the weight of said composition, 9% of $TiO_2$, 3% of $CaCO_3$, 0.25% of clay and 0.30% of carboxymethylcellulose.